# EUROPÄISCHE PATENTANMELDUNG

(11) **EP 1 219 257 A2**
(43) Veröffentlichungstag der Anmeldung: **03.07.2002**
(21) Anmeldenummer: 01128948.5
(22) Anmeldetag: 06.12.2001
(51) Int. Cl.: A61B 17/86

(54) **Schädelschraube**

(30) Priorität: 30.12.2000 DE 10065799
(71) Anmelder: REHAU AG + Co, 95111 Rehau (DE)
(72) Erfinder: Manthey, Jürgen, Dr., 95111 Rehau (DE); Gropp, Friedrich, Dr., 95111 Rehau (DE); Stendel, Rüdiger, 14163 Berlin (DE)

(57) **Zusammenfassung**

Schädelschraube mit einem hohlen Schaft, der einen mit einem Schraubengewinde versehenen Abschnitt zum Einschrauben in ein Loch in einer Schädeldecke und einen mehrkantigen Schraubenkopf aufweist, wobei der hohle Schaft zumindest im das Schraubengewinde aufweisenden Schaftabschnitt ein vom distalen Schraubenende her zugängliches mehrkantiges Innenprofil aufweist.

## Beschreibung

Die Erfindung betrifft eine Schädelschraube mit einem hohlen Schaft, der einen mit einem Schraubengewinde versehenen Abschnitt zum Einschrauben in ein Loch einer Schädeldecke und einen mehrkantigen Schraubenkopf aufweist.

Eine derartige Schädelschraube ist beispielsweise aus EP 0 195 455 B1 bekannt. Die dort gezeigte Schädelschraube zeigt einen hohlen Schaft, an dessen distalem Ende ein Gewindeabschnitt vorgesehen ist, über den die Schädelschraube in ein in die Schädeldecke des zu behandelnden Patienten gebohrtes Loch eingeschraubt wird. Durch den hohlen Schaft kann nach Setzen der Schädelschraube z. B. ein Katheter in das Zielgebiet, z. B. ein Blutgerinnsel oder dergleichen, geführt werden, über den dann das Blut oder etwaige andere Flüssigkeit, die sich angesammelt hat, abgesaugt werden.

Bei dem Einschrauben einer solchen Schädelschraube kann es nun mitunter vorkommen, dass die Schraube abreißt. Bei der aus EP 0 195 455 B1 bekannten Schädelschraube ist neben dem eigentlichen Schraubenkopf, mittels welchem unter Verwendung eines geeigneten Werkzeugs die Schädelschraube einzuschrauben ist, ein weiterer, im Wesentlichen unmittelbar an das Schraubengewinde anschließender Schraubenkopf vorgesehen, wobei die beiden benachbarten Schraubenköpfe über einen als Sollbruchstelle dienenden Schaftabschnitt voneinander beabstandet sind. Sofern diese bekannte Schädelschraube im Bereich dieses Schaftabschnittes reißt, besteht die Möglichkeit, den in den Schädel eingeschraubten Schraubenteil mittels des zweiten Schraubenkopfes zu greifen und herausschrauben zu können. Jedoch kommt es mitunter vor, dass die Schädelschraube im Bereich des Gewindeabschnitts selbst reißt, das heißt, auch der zweite Schraubenkopf der vorbekannten Schädelschraube reißt mit ab. In diesem Fall sind zum Entfernen der Schädelschraube aufwendige chirurgische Maßnahmen erforderlich.

Die genannten Probleme sind aber auch bei Schädelschrauben vorhanden, die lediglich einen Schraubenkopf aufweisen, auch hier besteht die Gefahr, dass die Schädelschraube im Bereich des Gewindeabschnitts abreißt.

Der Erfindung liegt damit das Problem zugrunde, eine Schädelschraube anzugeben, die auch in solchen Fällen ein einfaches Entfernen des in der Schädeldecke bei einem Schraubenriss verbliebenen Schraubenteils ermöglicht.

Zur Lösung dieses Problems ist bei einer Schädelschraube der eingangs genannten Art erfindungsgemäß vorgesehen, dass der hohle Schaft zumindest im das Schraubengewinde aufweisenden Schaftabschnitt ein vom distalen Schraubenende her zugängliches mehrkantiges Innenprofil aufweist.

Das mehrkantige Innenprofil, zweckmäßigerweise eine Sechskantprofil, kann auch bei einem Schraubenriss im Bereich des Schraubengewindes über ein geeignetes Werkzeug, z. B. einen Inbusschlüssel, gegriffen und der Schraubenrest problemlos aus dem Schädel herausgeschraubt werden, ohne dass irgendwelche chirurgischen Maßnahmen hierfür erforderlich wären. Das mehrkantige Innenprofil, das vom distalen Schraubenende her zweckmäßigerweise in jedem Fall, also nicht nur bei einem Abriss der Schraube zugänglich ist, sollte sich vorzugsweise längs des gesamten das Schraubengewinde aufweisenden Schaftabschnitts erstrecken. Der Umstand, dass das mehrkantige Innenprofil auch bei unbeschädigter Schädelschraube vom distalen Ende her zugänglich ist, bietet ferner die Möglichkeit, die Schädelschraube unter Verwendung des mehrkantigen Innenprofils im Schädel festzuschrauben, was mitunter dann zweckmäßig ist, wenn aufgrund diverser weiterer zur Behandlung erforderlicher Gerätschaften nur ein geringes Platzangebot zur Verfügung ist. Denn in diesem Fall besteht die Möglichkeit, über ein Werkzeug wie z. B. einen entsprechend profilierten Schraubendreher quasi in Verlängerung der Schädelschraube an dieser angreifen zu können, und nicht von der Seite, wie dies bei einem Schraubenkopf der Fall ist.

Eine weitere zweckmäßige Erfindungsausgestaltung sieht vor, dass der Gewindeabschnitt sich zum proximalen Ende des Schaftes hin verjüngt. Der Gewindeabschnitt bzw. der ihn aufweisende Schaftabschnitt besitzt demgemäß eine vorteilhafte kegelstumpfartige Form, das heißt, die ersten Gewindegänge des Gewindeabschnitts weisen einen schmäleren Durchmesser auf als die Nachfolgenden. Dies bewirkt mit besonderem Vorteil, dass diese ersten Gewindegänge relativ leicht im Schädelknochen greifen und das anfängliche Einschrauben sehr leicht geht, wobei sich der Kraftaufwand zunehmend verstärkt, je weiter die Schädelschraube eingeschraubt wird. Die Gewindegänge mit großem Durchmesser führen zu einer sehr guten Abdichtung der Schädelschraube zum Schädelknochen hin. Die Verwendung eines zusätzlichen kostenintensiven und verlierbaren Dichtelementes entfällt. Ein weiterer Vorteil ist, dass während des Einschraubens nicht kontinuierlich mit großer Kraft zu schrauben ist, wie dies bei normalen "zylindrischen" Gewindeabschnitten der Fall wäre und was bei hohem Kraftaufwand viel eher zu einem Abreißen führt.

Schließlich kann erfindungsgemäß vorgesehen sein, dass am distalen Ende des Schafts ein Koppelabschnitt zum Ankoppeln eines Drittgegenstandes vorgesehen ist. Wie beschrieben besteht bei einer solchen Schädelschraube die Möglichkeit, z. B. einen Katheter einzuführen, der in diesem Fall an dem Koppelabschnitt angekoppelt bzw. mittels des Koppelabschnitts gehaltert werden kann. Dieser Koppelabschnitt kann z. B. als Lueranschluss ausgebildet sein. Eine zweckmäßige Alternative hierzu sieht vor, dass der Koppelabschnitt als ein zweiter Gewindeabschnitt ausgebildet ist, auf den eine Überwurfmutter aufgeschraubt wird, über die der Katheter geklemmt werden kann. In diesem Zusammenhang ist es zweckmäßig, wenn eine vom distalen Ende her in den Schaft eingebrachte Sackbohrung zur Aufnahme eines Dichtelements, z. B. einer Silikondichtscheibe oder eines Silikondichtrings vorgesehen ist. Dieses Dichtelement wird mittels der Überwurfmutter, die einen entsprechenden, in die Sackbohrung eingreifenden Ringbund aufweist, beim Aufschrauben auf den zweiten Gewindeabschnitt gequetscht und kann sich so dichtend an den eingeschobenen Katheter anlegen.

Eine weitere Erfindungsalternative sieht vor, den Koppelabschnitt als mit einem Innengewinde versehene Sackbohrung auszubilden, in die ein entsprechendes, mit einem Außengewinde versehenes Gegenstück eingeschraubt werden kann, über welches beispielsweise der Katheter klemmbar ist.

Zur Begrenzung des Einschraubewegs kann die Schädelschraube nach einer ersten Erfindungsausgestaltung derart ausgebildet sein, dass sich der Schraubenkopf unmittelbar an den in den Schädelknochen einzuschraubenden Gewindeabschnitt anschließt. Das heißt, der Schraubenkopf selbst wird mit seiner dem Schädel zugewandten Seite direkt auf die Schädelaußenseite aufgeschraubt. Alternativ dazu besteht die Möglichkeit, einen an den in den Schädel einzuschraubenden Gewindeabschnitt anschließenden, im eingeschraubten Zustand an der Schädelaußenseite anliegenden Anlagebund oder dergleichen vorzusehen, der von dem Schraubenkopf beabstandet ist. Dieser Anlagebund kann zweckmäßigerweise als zweiter mehrkantiger Schraubenkopf ausgebildet sein, wie dies in ähnlicher Weise bei der Schädelschraube gemäß EP 0 195 455 B1 der Fall ist. Dieser zweite Schraubenkopf bietet, sofern die Schraube oberhalb dieses Schraubenkopfes abreißt, gleichermaßen die Möglichkeit, das verbliebene Schraubenteil herauszuschrauben. Alternativ kann der Anlagebund auch als Ringflansch ausgebildet sein. Der Anlagebund und der Schraubenkopf, der zum Einschrauben der Schädelschraube dient, können dabei über einen als Sollbruchstelle ausgebildeten Schaftabschnitt voneinander beabstandet sein.

Die Schädelschraube selbst kann aus Kunststoff oder Metall gefertigt sein. Als Kunststoff kommt z. B. PEEK (Polyetheretherketon) zum Einsatz, als Metalle können z. B. Edelstahl oder Titan verwendet werden. Besonders zweckmäßig ist es, wenn die Schädelschraube aus einem Metall ist, das bei einer Strahlenuntersuchung des Schädels nicht abgebildet wird, so dass artefaktfreie aussagekräftige Bilder erhalten werden. Dies ist z. B. bei einer Schädelschraube aus PEEK gewährleistet.

In einer weitern vorteilhaften Ausgestaltungsform ist die erfindungsgemäße Schädelschraube aus einem biologisch abbaubaren Kunststoff hergestellt.

Weitere Vorteile, Merkmale und Einzelheiten der Erfindung ergeben sich aus den im Folgenden beschriebenen Ausführungsbeispielen sowie anhand der Zeichnungen. Dabei zeigen:
- Fig. 1: eine erfindungsgemäße Schädelschraube einer ersten Ausführungsform in einer Schnittdarstellung,
- Fig. 2: die Schraube aus Fig. 1 in einer Aufsicht,
- Fig. 3: eine zweite erfindungsgemäße Ausführungsform einer Schädelschraube in einer Seitenschnittansicht,
- Fig. 4: eine Aufsicht auf die Schädelschraube aus Fig. 3,
- Fig. 5: eine Seitenansicht im Schnitt einer erfindungsgemäßen dritten Ausführungsform einer Schädelschraube, und
- Fig. 6: eine Aufsicht auf die Schädelschraube aus Fig. 5.

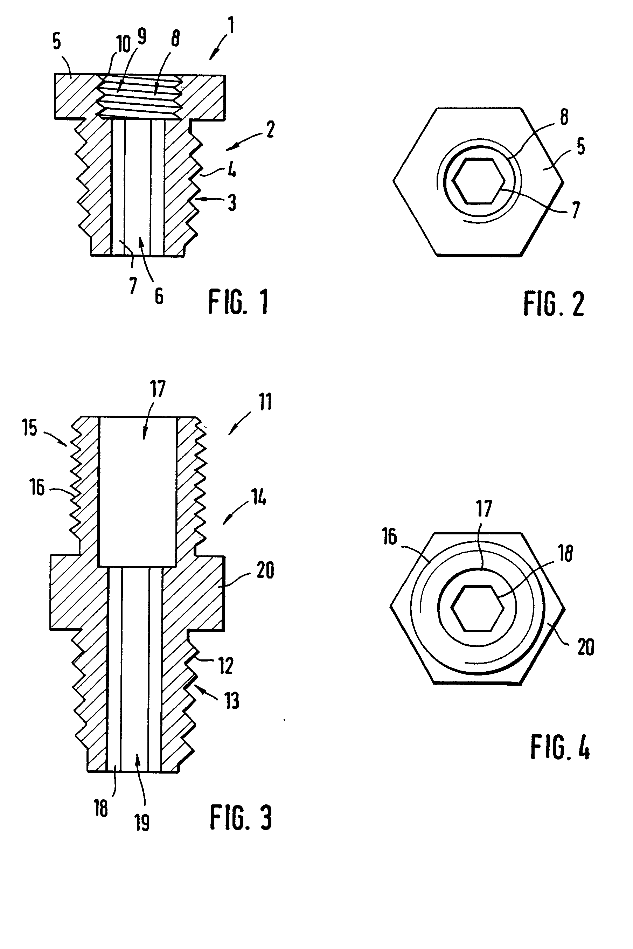

Fig. 1 zeigt eine erfindungsgemäße Schädelschraube 1 einer ersten Ausführungsform im Schnitt. Die erfindungsgemäße Schädelschraube 1 besteht aus einem Schaft 2, der einen Schaftabschnitt 3 aufweist, an dem ein Schraubengewinde 4 vorgesehen ist. An den Schaftabschnitt 3 schließt sich ein Schraubenkopf 5 an, der im gezeigten Ausführungsbeispiel, siehe Fig. 2, einen sechseckigen Querschnitt besitzt.

Der Schaft 2 ist innen hohl, wobei die zentrale Innendurchbrechung 6 im Bereich des Schaftabschnitts 3 ein mehrkantiges Innenprofil 7 aufweist, das, siehe Fig. 2, im gezeigten Ausführungsbeispiel als Sechskantprofil ausgebildet ist. Das Innenprofil 7 erstreckt sich über den gesamten Schaftabschnitt 3. Der Schraubenkopf 5 wiederum weist ebenfalls eine Durchbrechung in Form der Sackbohrung 8 auf, die, siehe Fig. 2, etwas größer bemessen ist als die zentrale Innendurchbrechung 6. Über den hohlen Schaft ist es nun möglich, nach Setzen der Schädelschraube 1 einen Katheter oder dergleichen ins Schädelinnere einzuführen.

Zum Setzen der Schädelschraube 1 wird diese mit dem Gewindeabschnitt 3 in eine entsprechende Bohrung in der Schädeldecke eingeschraubt. Um das Einschrauben zu erleichtern und gleichzeitig eine sichere Abdichtung zu erzielen verjüngt sich der Gewindeabschnitt 3 konisch oder kegelstumpfartig zum freien, proximalen Ende des Schafts hin. Dies führt dazu, dass die ersten Gewindezüge relativ leicht in das Knochenmaterial eingeschraubt werden können, wohingegen mit zunehmendem Einschraubweg die im Durchmesser breiteren Gewindezüge eingeschraubt werden, was einen höheren Kraftaufwand erfordert und zu einer noch besseren Abdichtung führt. Das Einschrauben wird mittels eines Werkzeugs, z. B. eines Sechskantschraubenschlüssels bewerkstelligt, der am Schraubenkopf 5 angreift.

Für den Fall, dass nun der Schraubenkopf 5 aus welchen Gründen auch immer abreißt und der Schaftabschnitt 3 bereits etwas in den Schädel eingeschraubt ist, so dass im Schädel ein relativ fest verankerter Schraubenrest zurückbleibt, kann dieser auf einfache Weise dadurch entfernt werden, dass mit einem geeigneten Werkzeug, z. B. einem Inbusschlüssel, in das Innenprofil 7 gegriffen und der Schraubenrest hierüber aus dem Schädel herausgeschraubt wird. Ein aufwendiger chirurgischer Eingriff ist damit nicht mehr erforderlich.

Weiterhin ist im Bereich der schraubenkopfseitigen Durchbrechung 8 ein Kopplungsabschnitt 9 vorgesehen, der ein Innengewinde 10 aufweist. In dieses Innengewinde kann z. B. ein zylindrisches, mit einem Außengewinde versehenes Klemmstück eingeschraubt werden, das den durch die Schädelschraube 1 durchgeschobenen Katheter nach Positionieren desselben klemmt. Dabei ist es auch denkbar, zur Verbesserung der Klemmung in die Durchbrechung 8 ein Dichtelement, z. B. einen Silikondichtring einzulegen, der mittels des einzuschraubenden Teils gequetscht wird und sich so dichtend an den Katheter anlegt.

Fig. 3 zeigt eine weitere Ausführungsform einer Schädelschraube 11. Diese entspricht im Aufbau insoweit der aus Fig. 1, jedoch ist an der dem mit dem Schraubengewinde 12 versehenen Schaftabschnitt 13 gegenüberliegenden Seite des Schafts 14 ein Koppelabschnitt 15 vorgesehen, der ein als Außengewinde ausgebildetes Schraubengewinde 16 aufweist. Über die zentrale Sackbohrung 17, die in Verlängerung der das mehrkantige Innenprofil 18 aufweisenden zentralen Durchbrechung 19 ausgebildet ist, kann ebenfalls ein Katheter oder dergleichen zugeführt werden, der z. B. mittels einer Überwurfmutter, die auf das Schraubengewinde 16 aufgeschraubt wird, geklemmt wird. Natürlich kann auch hier wiederum ein Silikondichtring in die Sackbohrung 17 eingebracht werden, der z. B. mittels eines an der Überwurfmutter vorgesehenen Ringbundes nach Aufschrauben der Überwurfmutter gequetscht wird und sich an den Katheter anlegt.

Bei dieser Ausführung ist lediglich ein Schraubenkopf 20 vorgesehen (siehe Fig. 4), wobei auch hier ein problemloses Ausschrauben im Falle eines Schraubenrisses aus dem Schädel unter Eingriff eines geeigneten Werkzeugs in das sechskantige Innenprofil 18 möglich ist.

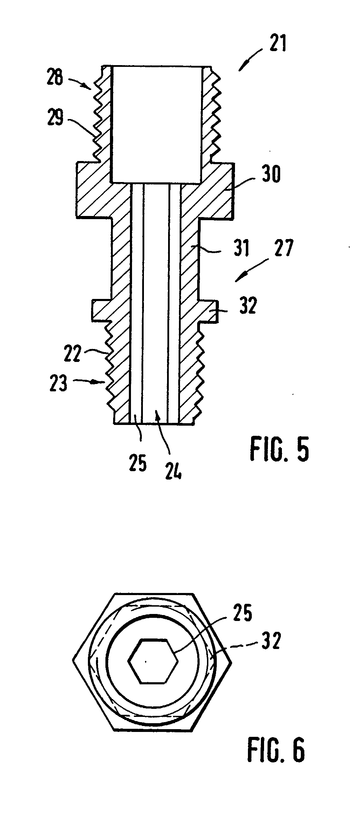

Die Fig. 5 und 6 zeigen schließlich eine dritte Ausführungsform einer Schädelschraube 21. Bei dieser Ausführungsform ist ebenfalls ein sich konisch verjüngender, mit einem Schraubengewinde 22 versehener Schaftabschnitt 23 vorgesehen, wobei auch hier eine zentrale Durchbrechung 24 mit einem Sechskant-Innenprofil 25 (s. Fig. 6) diesen Schaft 27 durchsetzt.

An der gegenüberliegenden Seite des Schafts 27 ist wiederum ein Koppelabschnitt 28 mit einem Außengewinde 29 vorgesehen, auf den wie bereits zur Ausführungsform nach Fig. 3 beschrieben eine Überwurfmutter aufgeschraubt werden kann.

Im Unterschied zur Ausführungsform nach Fig. 3 ist hier ein Schraubenkopf 30 vorgesehen, der unmittelbar unter dem Schraubengewinde 29 des Koppelabschnitts 28 ausgebildet ist. Ferner ist über einen Schaftabschnitt 31 beabstandet ein unmittelbar an das Schraubengewinde 22 anschließender Anlagebund 32 vorgesehen, der, siehe Fig. 6, ebenfalls als Sechskant-Schraubenkopf ausgebildet ist. Dieser Anlagebund 32, hier in Form des Schraubenkopfs, dient dazu, eine Schädelschraube 21, die im Bereich des Schaftabschnitts 31, der gegebenenfalls auch als Sollbruchstelle ausgebildet sein kann, abgerissen ist noch aus dem Schädel herausschrauben zu können, da er mit einem Werkzeug oder dergleichen gegriffen werden kann. Für den Fall, dass die Schädelschraube 21 jedoch im Bereich des Schaftabschnitts 23 reißt, kann die Entfernung über das sechskantige Innenprofil 25 erfolgen. Auch hier kann zu Dichtzwecken ein Dichtelement ín die obige Sackbohrung eingelegt werden.

Sämtliche Schädelschrauben 1, 11 und 21 sind zweckmäßigerweise aus einem Material gefertigt, das in einem Strahlungsbild, das vom Patienten aufgenommen wird, z. B. einer Röntgenaufnahme, einer Magnetresonanzaufnahme oder einer Computertomographieaufnahme, nicht sichtbar ist. Hier bietet sich insbesondere ein PEEK-Kunststoff an. Daneben besteht auch die Möglichkeit, die Schrauben aus Edelstahl oder Titan zu fertigen.

## Patentansprüche

1. Schädelschraube mit einem hohlen Schaft, der einen mit einem Schraubengewinde versehenen Abschnitt zum Einschrauben in ein Loch in einer Schädeldecke und einen mehrkantigen Schraubenkopf aufweist, **dadurch gekennzeichnet, dass** der hohle Schaft (2, 14, 27) zumindest im das Schraubengewinde (4, 12, 22) aufweisenden Schaftabschnitt (3, 13, 23) ein vom distalen Schraubenende her zugängliches mehrkantiges Innenprofil (7, 18, 25) aufweist.

2. Schädelschraube nach Anspruch 1, **dadurch gekennzeichnet, dass** sich das mehrkantige Innenprofil (7, 18, 25) den gesamten das Schraubengewinde (4, 12, 22) aufweisenden Schaftabschnitt (3, 13, 23) durchsetzt.

3. Schädelschraube nach Anspruch 1 oder 2, **dadurch gekennzeichnet, dass** das mehrkantige Innenprofil (7, 18, 25) ein Sechskantprofil ist.

4. Schädelschraube nach einem der vorangehenden Ansprüche, **dadurch gekennzeichnet, dass** der das Schraubengewinde (4, 12, 22) aufweisende Schaftabschnitt (3, 13, 23) sich zum proximalen Ende des hohlen Schafts (2, 14, 27) hin verjüngt.

5. Schädelschraube nach einem der vorangehenden Ansprüche, **dadurch gekennzeichnet, dass** am distalen Ende des Schafts (3, 13, 23) ein Koppelabschnitt (9, 15, 28) zum Ankoppeln eines Drittgegenstands vorgesehen ist.

6. Schädelschraube nach Anspruch 5, **dadurch gekennzeichnet, dass** der Koppelabschnitt (9, 15, 28) als Lueranschluss ausgebildet ist.

7. Schädelschraube nach Anspruch 5, **dadurch gekennzeichnet, dass** der Koppelabschnitt (9, 15, 28) als ein zweites Schraubengewinde (10, 16, 29) ausgebildet ist.

8. Schädelschraube nach Anspruch 7, **dadurch gekennzeichnet, dass** eine vom distalen Ende in den Schaft (2, 14, 27) eingebrachte Sackbohrung (8, 17) zur Aufnahme eines Dichtelements vorgesehen ist.

9. Schädelschraube nach Anspruch 5, **dadurch gekennzeichnet, dass** der Koppelabschnitt (9, 15, 28) als mit einem Innengewinde (10) versehene Sackbohrung (8) ausgebildet ist.

10. Schädelschraube nach einem der vorangehenden Ansprüche, **dadurch gekennzeichnet, dass** ein an den in den Schädel einzuschraubenden Schraubengewinde (4, 12, 22) anschließender, im eingeschraubten Zustand an der Schädelaußenseite anliegender Anlagebund (32) o.dgl. vorgesehen ist, der von dem Schraubenkopf (30) beabstandet ist.

11. Schädelschraube nach Anspruch 10, **dadurch gekennzeichnet, dass** der Anlagebund (32) als zweiter mehrkantiger Schraubenkopf ausgebildet ist.

12. Schädelschraube nach Anspruch 10, **dadurch gekennzeichnet, dass** der Anlagebund (32) als Ringflansch ausgebildet ist.

13. Schädelschraube nach einem der Ansprüche 10 bis 12, **dadurch gekennzeichnet, dass** der Anlagebund (32) und der Schraubenkopf (30) über einen als Sollbruchstelle ausgebildeten Schaftabschnitt (31) voneinander beabstandet sind.

14. Schädelschraube nach einem der vorangehenden Ansprüche, **dadurch gekennzeichnet, dass** sie aus Kunststoff oder Metall ist.

15. Schädelschraube nach Anspruch 14, **dadurch gekennzeichnet, dass** sie aus einem biologisch abbaubaren Kunststoff ist.

16. Schädelschraube nach einem der vorangehenden Ansprüche, **dadurch gekennzeichnet, dass** sie aus einem Material ist, das bei einer Strahlenuntersuchung des Schädels nicht abgebildet wird.
